# EUROPEAN PATENT APPLICATION

(11) **EP 2 392 318 A1**
(43) Date of publication of application: **07.12.2011**
(21) Application number: 10382135.1
(22) Date of filing: 21.05.2010
(51) Int. Cl.: A61K 9/20, A61K 9/28, A61K 31/4178

(54) **A pharmaceutical controlled release composition of losartan**

(71) Applicant: Laboratorios Liconsa, S.A., 08028 Barcelona (ES)
(72) Inventor: Sereno Guerra, Antonio, C-1820, MELSBROECK (BE); Loeches Blas, David, 28806, ALCALÁ DE HENARES (Madrid) (ES); Varas Fernández-Molina, Roberto, 19200, AZUQUECA DE HENARES (Guadalajara) (ES); Del Cura Medina, Estíbaliz, 28043, MADRID (ES); Rizo Martínez, José Miguel, 28043, MADRID (ES)
(74) Representative: Markvardsen, Peter

(57) **Abstract**

The present invention relates to a controlled release pharmaceutical composition of losartan which comprises a gastric retained core with losartan and an amount comprised between 5 and 25% by weight of hydroxypropyl methylcellulose as a unique controlled release polymer, and an active immediate release layer containing losartan, to its preparation process and its use for the prophylaxis and treatment of hypertension.

## Description

### FIELD OF THE INVENTION

The present invention relates to a pharmaceutical controlled release composition of losartan, to its preparation process and its use for the prophylaxis and/or treatment of hypertension.

### BACKGROUND ART

Losartan is the International Nonproprietary Name of 2-butyl-4-chloro-1-[[2'-(1H-tetrazol-5-yl)[1,1'-biphenyl]-4-yl]methyl]-1H-imidazole-5-methanol. Losartan is a non-peptide angiotensin II receptor antagonist (Type AT₁) and its structure corresponds to formula (I):

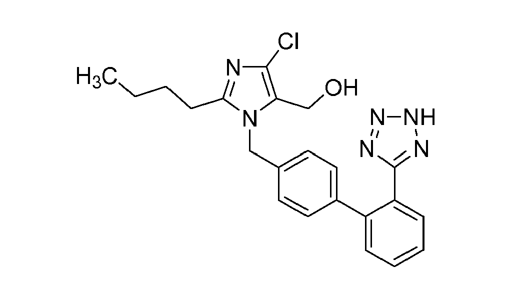

Losartan is marketed as potassium losartan which is used in regulating hypertension alone or in combination with other antihypertensive agents, including diuretics. It is also indicated to reduce stroke and left ventricular hypertrophy in hypertensive patients. Losartan also show effects in the prevention and treatment of renal failure, myocardial infraction, arrhythmia, heart failure and diabetic complications such as nephropathy in type II diabetic patients, and in the prevention of atherosclerosis and cardiovascular diseases. Losartan is commercially available as tablets for oral administration containing from 25 mg to 100 mg of the active ingredient, alone or in combination with hydrochlorothiazide. The typically dosing regimens are either once-daily or twice-daily.

Losartan after being orally administered undergoes substantial first-pass metabolized by cytochrom P450 enzymes. It is partly converted into an active metabolite that seems to be responsible for most of the angiotensin II receptor antagonism of losartan treatment. About 14% of the orally-administered dose of losartan is converted into this active metabolite in normal subjects. The terminal half-life of losartan and its active metabolite is about 2 hours and 6-9 hours respectfully, and their median maximum concentration is about 1 hour and 3-4 hours respectfully.

Losartan is readily orally absorbed in about 33% in the gastrointestinal tract, particularly in the upper parts of the small intestine where its absorption windows is located, otherwise losartan and its active metabolite are evacuated with the intestinal transit without being correctly absorbed.

Epidemiologic studies have shown that in patients with hypertension, blood pressure follows a highly reproducible circadian pattern characterized by a decrease of the blood pressure during sleep, and a rapid increase in the early morning period. Therefore, the early morning period has been associated with both loss of blood pressure control and increased rates of myocardial infarction and stroke, especially during the first 4 to 6 hours after awakening (Cf. William B. White. "Clinical assessment of early morning blood pressure in patients with hypertension". Preventive Cardiology. 2007 Fall, vol.10, pp. 173-4).

It has been reported that due to the reduced half life of losartan in comparison with other antihypertensive agents, its antihypertensive effects during the first hours after awaking is low, in particular when losartan is administered once-daily in the morning (Cf. Takuya Nishimura et al. "Efficacy and duration of action of the four selective angiotensin II subtype 1 receptor blockers, losartan, candesartan, valsartan and telmisartan, in patients with essential hypertension determined by home blood pressure measurements", Clinical and Experimental Hypertension. 2005, vol. 6, pp. 477-489).

Besides, the above-mentioned immediate release dosing regimes provoke alterations of nervous system, particularly dizziness and gastrointestinal side-effects. These side-effects are caused by the increase of the amount of the active ingredient required to counteract the amount of losartan eliminated without being absorbed. Besides, the therapeutic plasmatic concentrations are difficult to be achieved, and the blood pressure is not controlled, increasing the risk of suffering from an infarction.

The International patent application WO 03/35039 discloses gastric retained losartan dosage forms that contain hydroxypropyl methylcellulose as a swellable hydrophilic polymer alone, or in combination with polyethylene oxide. These polymers swell to a size that it promotes the retention of the dosage form in the stomach during the digestive state, or "fed mode", increasing the time to reach the maximum concentration (Cₘₐₓ) in plasma and tissue (tₘₐₓ) prolonging the anti-hypertensive effect over 24 hours. The total amount of the hydrophilic polymers is about 50% by the weight of the composition, taking into account that when a mixture of both hydroxypropyl methylcellulose and polyethylene oxide is used, the weight ratio between them is 50:50. Disadvantageously, in the above-mentioned gastric retained losartan dosage forms more than 60% by weight of losartan is retained after 6 hours of being administered. Therefore, there is not a real blood pressure control during the following hours to the oral administration of the gastric retained dosage form, increasing the risk of suffering from an infarction.

From what is known in the art it is derived that there is still the need of providing pharmaceutical compositions that allows an effective absorption of losartan in the upper parts of the small intestine in a continuous way, having effective plasmatic concentration of losartan and its active metabolite over 24 hours.

### SUMMARY OF THE INVENTION

Inventors have found that a pharmaceutical composition which comprises a gastric retained core comprising a pharmaceutically effective amount of losartan and hydroxypropyl methylcellulose (HPMC) as a unique controlled release polymer, that is without any other controlled release polymer, and an immediate release layer comprising a pharmaceutically effective amount of losartan, has an appropriate dissolution profile which allows having effective plasmatic concentrations over 24 hours, and avoids the evacuation of the unabsorbed active ingredient by the intestinal transit. It is advantageous for the reduction of the risk of cardiovascular accidents, and for having a better control of blood pressure. Besides, the compositions of the present invention are also advantageous because of its extended timing uptake allowing a reduction of the dose intake, thereby increasing the acceptance by the patients of the oral administered posologies.

Thus, an aspect of the present invention refers to a pharmaceutical composition which comprises a gastric retained core, and an immediate release layer, where: the core comprises a pharmaceutically effective amount of losartan, or its pharmaceutically acceptable salts, and an amount of hydroxypropyl methylcellulose comprised between 5 and 25% by the weight of the composition as a unique controlled release polymer, together with one or more pharmaceutically acceptable excipients or carriers; and the immediate release layer comprises a pharmaceutically effective amount of losartan, or its pharmaceutically acceptable salts, together with one or more pharmaceutically acceptable excipients or carriers.

Another aspect of the present invention refers to a process for the preparation of the pharmaceutical composition as defined above, which comprises (a) mixing losartan or its pharmaceutically acceptable salts, with hydroxypropyl methylcellulose, a filler, and a binder, followed by adding a glidant and a lubricant; (b) mixing losartan or its pharmaceutically acceptable salts with a filler, a binder, followed by adding a lubricant; (c) compressing the mixture of step (a); and (d) compressing the mixture of step (b) over the resultant core of step (c) to yield a tablet.

Finally, another aspect of the present invention relates to the pharmaceutical composition as defined above for use in the prophylaxis and/or treatment of hypertension.

### BRIEF DESCRIPTION OF THE DRAWINGS

FIG. 1 shows the dissolution profile of losartan from the pharmaceutical compositions of Examples 1 (▲), 2(x), 3(0) and 4(■). Units of the scheme are as follows: %D is the percentage of losartan dissolved and t is time in minutes.
FIG. 2 shows the dissolution profile of losartan from the marketed immediate release pharmaceutical composition containing 100 mg of the active ingredient (Cozaar). Units of the scheme are as follows: %D is the percentage of losartan dissolved and t is time in minutes.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

A "pharmaceutically effective amount" of losartan or its salts refers to that amount which provides a therapeutic effect in various administration regimens.

A "pharmaceutically acceptable salt" of losartan refers to those salts which are, within the scope of medical judgement, suitable for use in contact with the tissues of humans and animals without excessive toxicity, irritation, allergic response, or other problem or complication, commensurate with a reasonable benefit/risk ratio.

The term "percentage (%) by weight" refers to the percentage of each ingredient of the pharmaceutical composition in relation to the total weight of the pharmaceutical composition.

The term "controlled release" or "controlled delivery" refer to a delivery of the drug at a predetermined rate and/or location according to the needs of the body and disease states for a definite time of period.

The term "gastric retained" refers to a delivery of the drug by dispersion throughout the polymer and has difficulty in diffusing out of the polymer matrix. Therefore, the biological fluid diffuses into the matrix and causes its outer polymer region to swell, allowing release of the drug entrapped inside the polymer at a predictable rate. The retained gastric dosage form contains hydrophilic polymers that swell to a size such that the dosage form is retained in the fed mode.

The term "hydrophilic polymer" refers to the partition coefficient P, which is the ratio of the equilibrium concentration of a compound in an organic phase to that in an aqueous phase. A hydrophilic compound has a P value less than 1.0, typically less than about 0.5, where P is the partition coefficient of the compound between octanol and water. Thus, the polymeric carriers herein used are compatible with aqueous fluids such as those present in the human body.

The term "polymer" refers to a molecule containing a plurality of covalently attached monomer units, and includes branched, dendrimeric, star, and linear polymers. The term also includes homopolymer and copolymers, as well as uncrosslinked polymers and from slightly to moderately or substantially crosslinked polymers.

The term "fed mode" or "fasted state" refers to the digestive mode of a normal digestive process. The fed mode is typically induced by the presence of food in the stomach giving rise to continuous and regular contractions per minute while the pylorus is partially open. The partial opening of the pylorus causes a sieving effect in which liquids and small particles flow continuously from the stomach into the intestine, while particles exceeding about 1 cm in size are retained for approximately 4 to 6 hours.

The term "ratio" refers to the relation of amount of losartan of the core and the amount of losartan in the immediate release film-coating needed to have effective amounts of the active ingredient.

The term "filler" refers to a material which is used to increase the bulk, improve consistency and improve the handle. Materials commonly used as fillers include, but are not limited to, dextrose, lactose, lactose monohydrate, fructose, mannitol, microcrystalline cellulose, starch, pregelatinized starch, powdered cellulose, silicied cellulose, sorbitol, sucrose and talc, or mixtures thereof.

The term "binder" refers to a material which imparts cohesiveness to powdered materials improving free-flowing qualities in the manufacture of the solid dosage form. Materials commonly used as binders include, but are not limited to, starch, gelatin, sugars, sodium alginate, hydroxypropyl cellulose, hydroxypropylmethylcellulose, hydroxyethylcellulose, methylcellulose, polyvinylpyrrolidone, sodium carboxymethylcellulose, and calcium carboxymethylcellulose, or mixtures thereof. When tablets are prepared by dry compression the binder used is microcrystalline cellulose.

The term "glidant" refers to a material which improves the flow characteristics of powder mixtures in the dry state. Materials commonly used as a glidant include, but are not limited to, magnesium stearate, colloidal silicon dioxide, precipitated silica, and talc, or mixture thereof.

The term "lubricant" refers to a material which prevent from adhesion of the tablet material to the surface of dies and punches, reduce inter-particle friction, facilitate the ejection of the tablets from the die cavity, and may improve the rate of flow of the tablet granulation. Materials commonly used as a lubricant include, but are not limited to, talc, alkaline earth salts of stearic acid such as magnesium and calcium stearate, stearic acid, glyceryl behanate, hydrogenated vegetable oils, glycerin palmitosteartae, stearyl fumarate, and polyethylene glycol, or mixture thereof.

As mentioned above, an aspect of the present invention refers to a pharmaceutical composition which comprises a gastric retained core where the amount of HPMC is comprised between 5 and 25% by the weight of the composition as a unique controlled release polymer, together with one or more pharmaceutically acceptable excipients or carriers, and an immediate release layer, where both layers comprises pharmaceutically effective amount of losartan, or its pharmaceutically acceptable salts, together with one or more pharmaceutically acceptable excipients or carriers.

The pharmaceutical composition of the present invention which comprises a core that the amount of HPMC is comprised between 5 and 25% by weight of the composition as a unique controlled release polymer and an active immediate release layer allow having an appropriate dissolution profile of the active ingredient maintaining effective plasmatic concentrations for over 24 hours.

The use of the hydrophilic polymer, HPMC, as a matrix former allows swelling the core upon absorption of the gastric fluid and the gradual erosion over a time period of hours. The erosion process is simultaneously initiated with the swelling process upon contact of the surface of the dosage form with the gastric fluid. Erosion reflects the dissolution of the polymer beyond the formation of the gel-solution interface, where the polymer has become sufficiently diluted that the active ingredient can be transported away from the dosage form by diffusion or convention.

The content of HPMC comprised between 5 and 25% by weight of the composition as a unique controlled release polymer in the core of the pharmaceutical composition of the present invention, allows a balance of these both processes, that is swelling and erosion, having an appropriate dissolution profile that maintain effective concentrations of the active ingredient for over 24h.

In comparison with the controlled release formulations of losartan described in the prior art, where the dissolution of the active ingredient begins about 5 hours after its oral administration, the pharmaceutical composition of the present invention allows the dissolution of losartan in a short period of time, due to the present of the active immediate release layer.

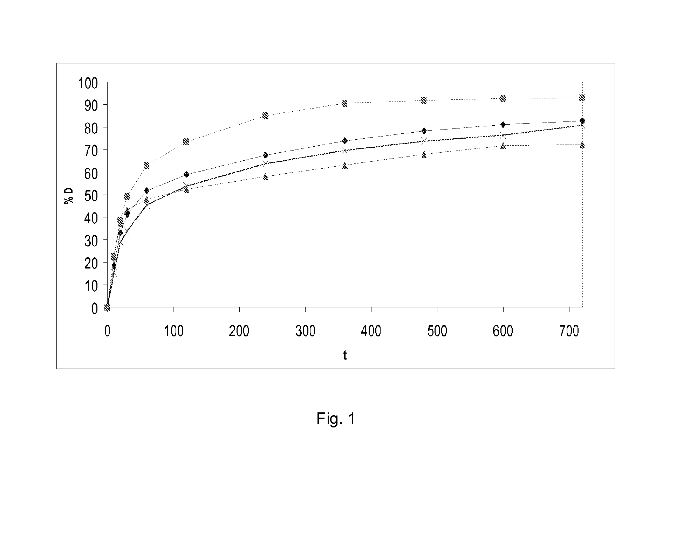

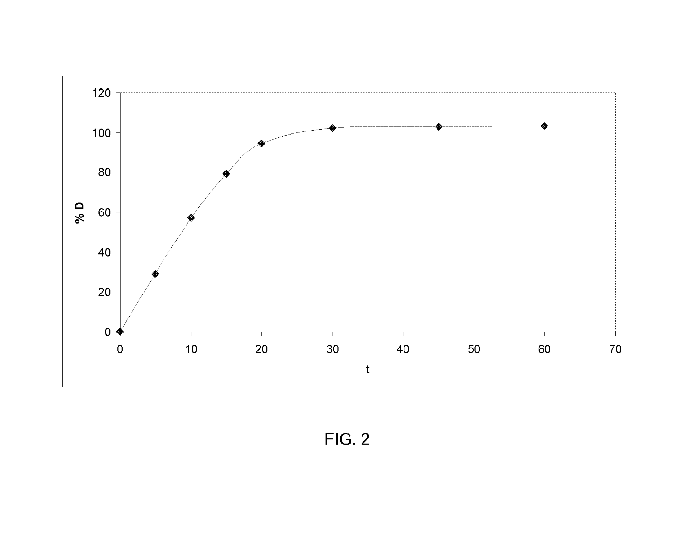

Otherwise, as it is shown in the comparative Example 6 table 1 (Fig. 1), the dissolution rate of losartan related to the immediate release layer of the present invention is slower than the dissolution rate of the marketed product Cozaar (Example 6 table 1 and Fig. 2). Thus, losartan in marketed tablets are dissolved in about 25 minutes, while in the composition of the present invention the content of the immediate release layer is dissolved in about 60 minutes. This is advantageous because this rapid dissolution rate of the active ingredient allows having effective amount of losartan in a short period of time after its administration; and at the same time, a smooth and slower dissolution profile which allows a better control of the absorption of losartan in its absorption window in the small intestine, avoiding the unaltered evacuation for the intestinal transit.

The target dissolution profile comprises that at least 35% by weight of losartan is retained in the dosage form after 1 hour and at least 70% by weight of losartan is dissolved from the dosage form after 12 hours of its oral administration.

Therefore, as it is shown in the Examples 1-4, the above required dissolution profile after oral administration of losartan is achieved by the pharmaceutical composition of the present invention (Example 6 Table 1 and Fig.1).

In a preferred embodiment, the above-mentioned composition of the present invention is further coated with an outer inert immediate release film-coating. This film-coating is formed by a water-soluble material to cover up the active ingredient possessing objectionable tastes or odors, or in protecting materials sensitive to oxidation. Examples of appropriate polymers to prepare the film-coating include soluble alkyl- or hydroxyalkylcellulose derivatives such as methylcellulose, hydroxymethylcellulose, hydroxyethylcellulose, hydroxypropylcellulose, low-substituted hydroxypropylcellulose (L-HPC), hydroxypropyl methylcellulose (HPMC), dextrins, soluble starches and starch derivatives, natural gums such as gum arabic, xanthans, alginates; and other water soluble film forming polymers such as polyvinylpyrrolidone, and polyethylene oxide, or mixture thereof. Preferably, the film-coating polymer is selected from the group consisting of low-substituted hydroxypropylcellulose (L-HPC), and hydroxypropyl methylcellulose (HPMC), or their mixtures. The film-coating polymer can be accompanied by plasticizers such as triethylcitrate (TEC), polyethylene glycol (PEG), cetyl and stearyl alcohol; surface-active agents such as sodium lauryl sulphate, polysorbate and poloxamer; pigments such as titanium dioxide, iron sesquioxide; lubricants such as talc, magnesium stearate or glyceril monostearate, and mixtures thereof.

In a particular embodiment the film-coating comprises hydroxypropylcellulose, and hydroxypropyl methylcellulose as a polymers, and titanium dioxide as a pigment.

Losartan can be in form of pharmaceutically acceptable salt of organic acids, inorganic acids or metals. Examples of appropriate inorganic acids include hydrochloric acid, hydrobromic acid, hydroiodic acid, nitric acid, perchloric acid, sulfuric acid or phosphoric acid. Examples of appropriate organic acids include methansulfonic acid, trifluoromethansulfonic acid, ethansulfonic acid, benzenesulfonic acid, p-toluenesulfonic acid, fumaric acid, citric acid, oxalic acid, acetic acid and maleic acid, among others. Examples of appropriate metals include alkaline or alkaline earth alkaline such as sodium, potassium, magnesium, and calcium, among others.

In a preferred embodiment of the present invention the pharmaceutically acceptable salt of losartan is potassium salt.

As it is mentioned above, the amount of HPMC in the gastric retained core is comprised between 5 and 25% by weight of the composition as a unique controlled release polymer. In a preferred embodiment, the amount of HPMC in the gastric retained core is comprised between 6 and 23% by weight of the composition. In a more preferred embodiment, the amount of HPMC in the gastric retained core is comprised between 7 and 20% by weight of the composition. In another more preferred embodiment, the amount of HPMC in the gastric retained core is comprised between 8 and 15% by weight of the composition. Preferably, the amount of HPMC in the gastric retained core is 13% by weight.

The pharmaceutical compositions of the present invention contain losartan both in the gastric retained core and in the active immediate-release layer. In a preferred embodiment the ratio between the amount of losartan in the core and the amount of losartan in the immediate release layer is comprised between 30:70 and 70:30. In a more preferred embodiment, the weight ratio between the amount of losartan in the core and the amount of losartan in the immediate release layer is comprised between 40:60 and 60:40. Preferably, the weight ratio between the amount of losartan in the core and the amount of losartan in the immediate release layer is 50:50. The above-mentioned weight ratio contributes to ensure that losartan is released from the composition of the present invention with the target dissolution profile, in order to achieve and maintain effective concentrations for over 24 hours.

It is known that losartan and its active metabolite have linear pharmacokinetic in a dose range comprised between 10 and 200 mg. In a preferred embodiment of the present invention the total amount of losartan is comprised between 50 and 200 mg. In a more preferred embodiment the total amount of losartan is comprised between 70 and 150 mg. Preferably, the total amount of losartan is 100 mg.

The pharmaceutical composition of the present invention can contain additional pharmaceutical excipients. The excipients must be selected from those that do not degrade the active ingredient in order to prepare the orally administrable pharmaceutical composition of the invention.

The pharmaceutical compositions defined above comprise pharmaceutically acceptable excipients or carriers appropriate for their oral administration.

In a preferred embodiment, the gastric retained core comprises a filler, a glidant, a lubricant, a binder, or their mixtures. Besides, the immediate release layer could be formed by excipients which help the formation of the active layer around the gastric retained core. Preferably, the immediate release layer comprises a filler, a lubricant, a binder, or their mixtures.

In a preferred embodiment, the gastric retained core of the composition of the present invention comprises: 5-30% by weight of potassium losartan; 5-25% by weight of hydroxypropyl methylcellulose; 15-50% by weight of filler; 0.1-10% by weight of lubricant; 0.1-5% by weight of glidant; and 2-20% by weight of binder; and the immediate release layer comprises: 5-20% by weight of potassium losartan; 2-30% by weight of filler; 5-20% by weight of binder; and 0.1-5% by weight of lubricant, being the sum of components of the composition 100%.

In another preferred embodiment, the gastric retained core of the composition of the present invention comprises: 8-20% by weight of potassium losartan; 8-15% by weight of hydroxypropyl methylcellulose; 15-45% by weight of filler; 0.1-5% by weight of lubricant; 0.1-2% by weight of glidant; and 8-15% by weight of binder; and the immediate release layer comprises: 8-15% by weight of potassium losartan; 9-23% by weight of filler; 8-15% by weight of binder; and 0.1-2% by weight of lubricant, being the sum of components of the composition 100%.

Preferably, the gastric retained core of the composition of the present invention comprises: 12.6% by weight of potassium losartan; 12.8% by weight of hydroxypropyl methylcellulose; 22.7% by weight of mannitol; 0.7% by weight of magnesium stearate; 0.2% by weight of colloidal silicon dioxide; and 10% by weight of microcrystalline cellulose; and the immediate release layer comprises: 12.6% by weight of potassium losartan; 13.2% by weight of microcrystalline cellulose; 5.2% by weight of pregelatinized maize starch; 6.4% by weight of lactose; and 0.2% by weight of magnesium stearate.

Typically, losartan is orally administered as a monotherapy for the treatment of hypertension at doses of 50, 100 and 150 mg once-daily or twice-daily. It is shown that 150 mg dose give not greater effect than 50-100mg. Besides, physiopathologic studies have demonstrated that the frequency of administration of losartan is more relevant than the administered amount to the control of the effectively of the treatment (cf. Alan H. Gradman et al. "A randomized, placebo-controlled, double-blind, parallel study of various doses of losartan potassium compared with enalapril maleate in patients with essential hypertension". Hypertension, 1995, vol. 25, pp. 1345-1350; Domenic A. Sica et al. "Clinical pharmacokinetics of losartan", Clinical Pharmacokinet, 2005, vol. 44, pp. 797-814). Thus, twice-daily dosing at 50-100 mg/day gave consistently larger responses than once-daily dosing at the same total dose, and a twice-daily regimen is recommended to maintain the effective dose during 24 hours.

In preferred embodiment, the pharmaceutical composition of the present invention is administered once-daily. This regimen is advantageous because allows a better control of the blood pressure and cardiovascular disorders, a simplicity of the oral administered posologies, together with a better acceptance and compliment of the treatment by the patient.

The pharmaceutical composition of the present invention can be prepared by conventional techniques that are well known in the pharmaceutical industry. The gastric retained core and the immediate release layer can for example be fabricated by direct compression in punches and dies fitted to a rotary tablet press, or by ejection or compression molding, granulation followed by compression, or the formation of a paste that is then extruded into a mold or into an extrude which is then cut into short lengths. The outer inner immediate release film-coating can be applied as a coating over the immediate release layer spraying, dipping, or pan-coating.

Thus, the pharmaceutical compositions of the present invention can be prepared by a process which comprises: which comprises: (a) mixing losartan or its pharmaceutically acceptable salts, with hydroxypropyl methylcellulose, a filler, and a binder, followed by adding a glidant, and a lubricant; (b) mixing losartan or its pharmaceutically acceptable salts with a filler, a binder, followed by adding a lubricant; (c) compressing the mixture of step (a); and (d) compressing the mixture of step (b) over the resultant core of step (c) to yield a tablet. Thus, both compression steps of the gastric retained core and the immediate release layer is carried out by direct compression

In a preferred embodiment, the process as mentioned above further comprising coating the tablets of step (d) by spraying an aqueous suspension which comprises an immediate release film-coating polymer; and drying the film-coating layer formed.

As it has been mentioned, it is also part of the invention the pharmaceutical composition defined above for use in the prophylaxis and/or treatment of hypertension. This aspect could be also formulated as the use of the pharmaceutical composition as defined above for the preparation of a medicament for the prophylaxis and/or treatment of hypertension. It is also part of the invention a method for the prophylaxis and/or treatment of hypertension which comprises administering to a mammal in need of such prophylaxis and/or treatment, an effective amount of the pharmaceutical composition of the present invention.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### Example 1: Tablet composition is as follow:

| Phase | Composition | Weight % by phase | Weight % by tablet |
|---|---|---|---|
| Gastric retained Core | Mannitol (Perlitol 300DC) | 31.93 | 20.31 |
| | Potassium losartan | 17.54 | 11.16 |
| | Magnesium stearate | 1.05 | 0.67 |
| | Colloidal silicon dioxide (aerosil 200) | 0.35 | 0.22 |
| | Hydroxypropyl methylcellulose (Methocel K-15) | **35.09** | **22.32** |
| | Microcrystalline cellulose (comprecel 200) | 14.04 | 8.93 |
| Active immediate-release film-coating | Potassium losartan | 33.33 | 11.16 |
| | Microcrystalline cellulose (comprecel 200) | 35.00 | 11.72 |
| | pregelatinized maize starch | 13.97 | 4.68 |
| | Lactose (fast flow) | 17.00 | 5.69 |
| | Magnesium stearate | 0.70 | 0.23 |
| Outer inert immediate-release film-coating | Hydroxypropyl cellulose | 40.00 | 1.16 |
| | Hydroxypropyl methylcellulose (HPMC-606) | 40.00 | 1.16 |
| | Titanium dioxide | 20.00 | 0.58 |
| | Purified water | q.s | q.s |
| | TOTAL | 100 | 100 |

| | | | |
|---|---|---|---|
| "q.s" means "as needed"; Water removed after processing | | | |

### Example 2: Tablet composition is as follow:

| Phase | Composition | Weight % by phase | Weight % by tablet |
|---|---|---|---|
| Gastric retained Core | Mannitol (Perlitol 300DC) | 28.30 | 22.70 |
| | Potassium losartan | 21.28 | 12.61 |
| | Magnesium stearate | 1.28 | 0.76 |
| | Colloidal silicon dioxide (aerosil 200) | 0.43 | 0.25 |
| | Hydroxypropyl methylcellulose (Methocel K-15) | **21.70** | **12.86** |
| | Microcrystalline cellulose (comprecel 200) | 17.02 | 10.09 |
| Active immediate-release film-coating | Potassium losartan | 33.33 | 12.61 |
| | Microcrystalline cellulose (comprecel 200) | 35.00 | 13.24 |
| | pregelatinized maize starch | 13.97 | 5.28 |
| | Lactose (fast flow) | 17.00 | 6.43 |
| | Magnesium stearate | 0.70 | 0.26 |
| Outer inert immediate-release film-coating | Hydroxypropyl cellulose | 40.00 | 1.16 |
| | Hydroxypropyl methylcellulose (HPMC-606) | 40.00 | 1.16 |
| | Titanium dioxide | 20.00 | 0.58 |
| | Purified water | q.s | q.s |
| | TOTAL | 100 | 100 |

| | | | |
|---|---|---|---|
| "q.s" means "as needed"; * Water removed after processing | | | |

### Example 3: Tablet composition is as follow:

| Phase | Composition | Weight % by phase | Weight % by tablet |
|---|---|---|---|
| Gastric retained Core | Mannitol (Perlitol 300DC) | 44.68 | 26.48 |
| | Potassium losartan | 21.28 | 12.61 |
| | Magnesium stearate | 1.28 | 0.76 |
| | Colloidal silicon dioxide (aerosil 200) | 0.43 | 0.25 |
| | Hydroxypropyl methylcellulose (Methocel K-15) | 15.32 | 9.08 |
| | Microcrystalline cellulose (comprecel 200) | **17.02** | **10.09** |
| | Potassium losartan | 33.33 | 12.61 |
| Active immediate-release film-coating | Microcrystalline cellulose (comprecel 200) | 35.00 | 13.24 |
| | Pregelatinized maize starch | 13.97 | 5.28 |
| | Lactose (fast flow) | 17.00 | 6.43 |
| | Magnesium stearate | 0.70 | 0.26 |
| Outer inert immediate-release film-coating | Hydroxypropyl cellulose | 40.00 | 1.16 |
| | Hydroxypropyl methylcellulose (HPMC-606) | 40.00 | 1.16 |
| | Titanium dioxide | 20.00 | 0.58 |
| | Purified water* | q.s | q.s |
| | TOTAL | 100 | 100 |

| | | | |
|---|---|---|---|
| "q.s" means "as needed"; * Water removed after processing | | | |

### Example 4: Tablet composition is as follow:

| Phase | Composition | Weight % by phase | Weight % by tablet |
|---|---|---|---|
| Gastric retained Core | Mannitol (Perlitol 300DC) | 49.79 | 29.51 |
| | Potassium losartan | 21.28 | 12.61 |
| | Magnesium stearate | 1.28 | 0.76 |
| | Colloidal silicon dioxide (aerosil 200) | 0.43 | 0.25 |
| | Hydroxypropyl methylcellulose (Methocel K-15) | **10.21** | **6.05** |
| | Microcrystalline cellulose (comprecel 200) | 17.02 | 10.09 |
| Active immediate- release film- coating | Potassium losartan | 33.33 | 12.61 |
| | Microcrystalline cellulose (comprecel 200) | 35.00 | 13.24 |
| | pregelatinized maize starch | 13.97 | 5.28 |
| | Lactose (fast flow) | 17.00 | 6.43 |
| | Magnesium stearate | 0.70 | 0.26 |
| Outer inert immediate- release film- coating | Hydroxypropyl cellulose | 40.00 | 1.16 |
| | Hydroxypropyl methylcellulose (HPMC-606) | 40.00 | 1.16 |
| | Titanium dioxide | 20.00 | 0.58 |
| | Purified water* | q.s | q.s |
| | TOTAL | 100 | 100 |

| | | | |
|---|---|---|---|
| "q.s" means "as needed"; * Water removed after processing | | | |

### Example 5: Process for the manufacture of the tablets of Examples 1-4

### 5.1. Process for the manufacture of the tablets of Example 4

### A. Process for the manufacture of a mixture of the ingredients of the core (Mixture A)

All ingredients of the core were separately sieved throught a sieve of 600 µm. In a Cyclops mixer the mannitol, microcrystalline cellulose, potassium losartan, and hydroxypropyl methylcellulose were added, and the resultant mixture was blended during 30 min. Colloidal silicon dioxide to the above obtained mixture was incorporated, and the mixture was blended during 5 min. After that time, magnesium stearate was also incorporated, and the resultant mixture was blended during 5 min to obtain Mixture A.

### B. Process for the manufacture of the mixture of ingredients of the immediate release layer (Mixture B)

Step 1: Potassium losartan, lactose and magnesium stearate were separately sieved through a sieve of 600 µm.

Step 2: In a Cyclops mixer lactose and microcrystalline cellulose were added, and the resultant mixture was blended during 10 min. The resultant mixture of step 1 was removed from the mixer and stored in a polyethylene bag.

Step 3: In the Cyclops mixer potassium losartan and pregelatinized starch were successively added, and the resultant mixture was blended during 10 min. After that time, mixture of step 2 was added, and the resultant mixture was blended during 30 min. Magnesium stearate was finally incorporated, and blended during 5 min to obtain Mixture B.

### C. Compression

Mixture A and mixture B were compressed to provide tablets of potassium losartan whose core contains mixture A and the immediate release layer contains mixture B.

Tablets were compressed on a Fette 102i rotary tablet press. The rotor speed was 25.000 tablets per hour, and the compression force was about 1.3KN for mixture A and 10KN for mixture B. Obtained tablets had a median hardness of 100N and ≤1% of friability.

### D. process for the manufacture of film-coating tablets

The film-coating suspension was prepared by blending in water L-HPC and HPMC until dissolution, maintaining vigorously agitation during 10 min. Titanium dioxide was added to the obtained dissolution and blended during 30 min. Finally, the obtained suspension was settled for 1 hour with an agitation pressure of 0.5 ± 0.1 Bar.

The film-coating were performed using a coating pan, at feed air temperature of 40 °C. The sprayed flow rate is 20 g/min. The final total weight of obtained tablets is 393 mg ± 2.

### 5.2. Process for the manufacture of the tablets of Examples 1-3

Tablets of examples 1, 2 and 3 were prepared analogously to the tablets of the previous section (5.1.).

### Example 6: Dissolution profile.

The target dissolution profile requires that the pharmaceutical composition of the present invention is swelled and retained under the stomach conditions where losartan is dissolved, and that the therapeutic concentration is achieved in the upper parts of the small intestine for a period of time about 24 hours.

The dissolution test was carried out to a solution of 900 mL at 37°C and at 50 rpm using a Pharmatest (PTWS, Germany) as a dissolution apparatus II USP according to the conditions described in the USP30 Pharmacopoeia.

### Conditions of the dissolution bath

- Paddle speed: 50 rpm
- Temperature of dissolution medium: 37 °C ± 0.5 °C
- Media: phosphate Buffer pH 6.8 (250 ml tribasic phosphate 0.2M + 750 ml HCl 0.1N)
- Vessel volume: 500 mL

### Table 1. Dissolution profile

The dissolution profile of Examples 1-4, Cozaar (marketed immediate release tablets of 100 mg potassium losartan), are as follows:

| Time | Dissolved losartan (weight %) | | | | |
|---|---|---|---|---|---|
| (min) | **Example 1** | **Example 2** | **Example 3** | **Example 4** | **Cozaar** |
| 0 | 0(0) | 0(0) | 0(0) | 0(0) | 0(0) |
| 5 | - | - | - | - | 29.1(14) |
| 10 | 22.4(14) | 15.2(31) | 18.5(22) | 22.9(38) | 57.2(10) |
| 15 | - | - | - | - | 79.3 (8) |
| 20 | 37.3(14) | 29.0(34) | 33.0(16) | 38.7(27) | 94.6 (7) |
| 30 | 43.3(12) | 33.8(29) | 41.1(12) | 49.1(21) | 102.2(2) |
| 45 | - | - | - | - | 102.9(1) |
| **60** | **48.0(6)** | **45.3(14)** | **51.6(9)** | **63.1(16)** | **103(1)** |
| 120 | 52.4(4) | 53.8(12) | 58.6(8) | 73.4(15) | - |
| 240 | 58.1(3) | 63.8(10) | 67.5(6) | 85.0(8) | - |
| 360 | 63.0(4) | 69.6(8) | 73.8(5) | 90.5(5) | - |
| 480 | 67.9(6) | 73.5(7) | 78.2(5) | 91.7(4) | - |
| 600 | 71.6(6) | 76.3(6) | 81.1(4) | 92.5(3) | - |
| **720** | **72.2(3)** | **80.9(5)** | **82.6(3)** | **93.1(4)** | - |

Pharmaceutical composition of Example 1 contains 22.32% HPMC in the core.
Pharmaceutical composition of Example 2 contains 12.86% HPMC in the core.
Pharmaceutical composition of Example 3 contains 9.08% HPMC in the core.
Pharmaceutical composition of Example 4 contains 6.05% HPMC in the core.
Comparative pharmaceutical composition of Cozaar contains 100 mg of potassium losartan, and HPMC in tablets of immediate release dosage form.

The dissolution profile results in Table 1 show that the pharmaceutical composition of the invention (Examples 1-4) which comprises an amount of HPMC comprised between 5 and 25% by weight as a unique controlled release polymer in the gastric retained core and an active immediate release film-coating have the required dissolution profile. Thus, the dissolution of losartan comprises that at least 35% by weight of losartan is retained in the dosage form after 1 hour and at least 70% by weight of losartan is dissolved from the dosage form after 12 hours after being orally administered. Comparative pharmaceutical composition of Cozaar (not using HPMC as a controlled release polymer) gives rise to a different dissolution profile in comparison with the percentage of the weight of losartan which is dissolved by the immediate release layer. Thus, while at 1 hour 100% of losartan is dissolved in the reference example Cozaar, in the pharmaceutical composition of the present invention (Examples 1-4) at 30 min between 32-50% of losartan is dissolved (the 50% by weight of the total amount of losartan, which corresponds to the total amount of losartan that can be delivered from the immediate release film-coating layer).

### PRIOR ART REFERENCE MENTIONED IN THE APPLICATION

1. William B. White. "Clinical assessment of early morning blood pressure in patients with hypertension", Preventive Cardiology. 2007, vol.10, pp. 173-4.
2. WO 03/35039
3. Takuya Nishimura et al. "Efficacy and duration of action of the four selective angiotensin II subtype 1 receptor blockers, losartan, candesartan, valsartan and telmisartan, in patients with essential hypertension determined by home blood pressure measurements", Clinical and Experimental Hypertension. 2005, vol. 6, pp. 477-489.
4. Alan H. Gradman et al. "A randomized, placebo-controlled, double-blind, parallel study of various doses of losartan potassium compared with enalapril maleate in patients with essential hypertension". Hypertension, 1995, vol. 25, pp. 1345-1350.
5. Domenic A. Sica et al. "Clinical pharmacokinetics of losartan", Clinical Pharmacokinet, 2005, vol. 44, pp. 797-814.

## Claims

1. A pharmaceutical composition which comprises a gastric retained core, and an immediate release layer, wherein:
the core comprises a pharmaceutically effective amount of losartan, or a pharmaceutically acceptable salt thereof, and an amount of hydroxypropyl methylcellulose comprised between 5 and 25% by the weight of the composition as a unique controlled release polymer, together with one or more pharmaceutically acceptable excipients or carriers; and
the immediate release layer comprises a pharmaceutically effective amount of losartan, or a pharmaceutically acceptable salt thereof, together with one or more pharmaceutically acceptable excipients or carriers.

2. The pharmaceutical composition according to claim 1, further comprising an outer inert immediate release film-coating.

3. The pharmaceutical composition according to any of the claims 1-2, wherein the pharmaceutically acceptable salt of losartan is the potassium salt.

4. The pharmaceutical composition according to any of the claims 1-3, wherein the amount in the core of hydroxypropyl methylcellulose is comprised between 8 and 15% by the weight of the composition.

5. The pharmaceutical composition according to any of the claims 1-4, wherein the amount in the core of hydroxypropyl methylcellulose is 13% by the weight of the composition.

6. The pharmaceutical composition according to any of the claims 1-5, wherein the weight ratio between the amount of losartan in the core and the amount of losartan in the immediate release layer is comprised between 30:70 and 70:30.

7. The pharmaceutical composition according to claim 6, wherein the weight ratio between the amount of losartan in the core and the amount of losartan in the immediate release layer is 50:50.

8. The pharmaceutical composition according to any of the claims 1-7, wherein the total amount of losartan is comprised between 50 and 200 mg.

9. The pharmaceutical composition according to any of the claims 1-8, wherein the core comprises: a filler, a glidant, a lubricant, a binder, or mixtures thereof; and the immediate release layer comprises: a filler, a lubricant, a binder, or mixtures thereof.

10. The pharmaceutical composition according to claim 9, wherein the core comprises:
5-30% by weight of potassium losartan;
5-25% by weight of hydroxypropyl methylcellulose;
15-50% by weight of filler;
0.1-10% by weight of lubricant;
0.1-5% by weight of glidant; and
2-20% by weight of binder;
and the immediate release layer comprises:
5-20% by weight of potassium losartan;
2-30% by weight of filler;
5-20% by weight of binder; and
0.1-5% by weight of lubricant;
being the sum of components of the composition 100%.

11. The pharmaceutical composition according to claim 10, wherein the core comprises:
12.6% by weight of potassium losartan;
12.8% by weight of hydroxypropyl methylcellulose;
22.7% by weight of mannitol;
0.7% by weight of magnesium stearate;
0.2% by weight of colloidal silicon dioxide; and
10% by weight of microcrystalline cellulose;
and the immediate release layer comprises:
12.6% by weight of potassium losartan;
13.2% by weight of microcrystalline cellulose;
5.2% by weight of pregelatinized maize starch;
6.4% by weight of lactose; and
0.2% by weight of magnesium stearate.

12. The pharmaceutical composition according to any of the claims 1-11, wherein the pharmaceutical composition is administered once-daily.

13. A process for the preparation of the pharmaceutical composition as defined in any of the claims 1-11, which comprises:
(a) mixing losartan or a pharmaceutically acceptable salt thereof, with hydroxypropyl methylcellulose, a filler, and a binder, followed by adding a glidant, and a lubricant;
(b) mixing losartan or a pharmaceutically acceptable salt thereof with a filler, a binder, followed by adding a lubricant;
(c) compressing the mixture of step (a); and
(d) compressing the mixture of step (b) over the resultant core of step (c) to yield a tablet.

14. The process according to claim 13, further comprising coating the tablets of step (d) by spraying an aqueous suspension which comprises an immediate release film-coating polymer; and drying the film-coating layer formed.

15. The pharmaceutical composition as defined in any of the claims 1-12, for use in the prophylaxis and/or treatment of hypertension.
